# EUROPEAN PATENT APPLICATION

(11) **EP 3 896 430 A1**
(43) Date of publication of application: **20.10.2021**
(21) Application number: 20169908.9
(22) Date of filing: 16.04.2020
(51) Int. Cl.: G01N 21/64, C12Q 1/68, G01N 33/68

(54) **IN-VITRO METHOD AND DEVICE FOR DETECTING A TARGET NUCLEID ACID**

(71) Applicant: ICHORtec GmbH, 52159 Roetgen (DE)
(72) Inventor: Fedorych, Oleh, 52062 Aachen (DE)
(74) Representative: Fuchs Patentanwälte Partnerschaft mbB

(57) **Abstract**

We disclose an in-vitro method for detecting a target nucleic acid in a patient's sample, comprising the following steps: Bringing the sample in contact with a probe that is a molecular beacon labeled with a fluorescent dye and which can hybridize with the target, then irradiating the probe with light exciting the fluorescent dye, then observing the sample with a detector (15), and then, if the detector (15) detects light emitted by the probe, concluding that the target is in the sample.

In order to speed up detection of the emitted light, the probe is immobilized in a carrier element (7) prior to bringing it in contact with the sample , wherein the carrier element (7) is in contact with an optical element (8) which lets the emitted light pass to the detector (15).

## Description

The invention concerns an in-vitro method for detecting a target nucleic acid in a patient's sample, comprising the following steps: Bringing the sample in contact with a probe, that is a molecular beacon, is labeled with a fluorescent dye, and can hybridize with the target, then irradiating the probe with light exciting the fluorescent dye, then observing the sample with a detector, and then, if the detector detects light emitted by the probe, concluding that the target is in the sample.

Viral and bacterial infections of the human body represent a major global public health concern in the 21^{st} century. Appropriate treatment depends on a correct diagnosis, not only of the type of the infectious agent, but also of the load or degree of infection. Presently most diagnostic methods for detecting infections are based on the detection of the DNA of the infectious agent by polymerase chain reaction (PCR) techniques which usually take about 90 minutes to 3 hours. PCR allows to make a large number of copies of a nucleic acid sample, and thus allowing scientists to take a very small sample and amplify it to a large enough amount to study it in detail. PCR is a powerful tool widely used in in-vitro diagnostics. The method allows detecting the presence of individual RNA/DNA in a patient's sample which makes PCR more reliable in comparison with immunoassay (ELISA) or similar techniques. Several modifications of the PCR method were also developed, e.g. reverse transcription PCR (RT-PCR), real time PCR or quantitative PCR (qPCR).

However, a reliable detection of target nucleic acids within few minutes and outside of the laboratory is currently not possible with any available technique.

### Problem to be solved

Thus, the object of the present invention is to develop a device and a method for detecting target nucleic acids quickly and reliably. Since the detection is based on fluorescence measurement, the invention shall speed up detection of the emitted light.

### Solution

This object is solved by a method of claim 1, an analyzing device of claim 7, and a sampling unit of claim 12. Preferred embodiments are the subject-matter of the dependent claims.

In particular, the present invention concerns an in-vitro method for detecting a target nucleic acid in a patient's sample, comprising the following steps:
a. Bringing the sample in contact with a probe, that is a molecular beacon labeled with a fluorescent dye, and can hybridize with the target, then
b. Irradiating the probe with light exciting the fluorescent dye, then
c. Observing the sample with a detector, and then
d. If the detector detects light emitted by the probe, concluding that the target is in the sample,

*Characterized by,* prior to bringing in contact with the sample, immobilizing the probe in a carrier element, wherein the carrier element is in contact with an optical element which lets the emitted light pass to the detector.

As mentioned above, the method of the present invention comprises the step of prior to bringing in contact with the sample, immobilizing the probe in the carrier element, wherein the probe is a molecular beacon, and the carrier element is in contact with the optical element which lets the emitted light pass to the detector. The invention is based on the finding that, the emitted light of a low number of target-probe hybrid molecules, or even of one single such molecule can be detected when in the focus of the detector. The invention avoids false positive emission of non-hybridized probe molecules. The invention mechanically fixes the hybridized molecules in the focus of the detector and thus enables detection of each single hybrid, without amplification (e.g. PCR or any other thermal amplification using polymerase).

As mentioned above, step (a) of the method of the present invention is based on labeling a nucleic acid contained in a patient's sample (= target nucleic acid) with a probe. According to the present invention this probe is a molecular beacon. This probe (= molecular beacon) is immobilized to a carrier element and hybridizes to the target nucleic acid under appropriate conditions. It is not necessary to extract the target nucleic acid (RNA or DNA) in advance since the method is very sensitive and is able to detect less than 10 copies. However, depending on the sample obtained, extraction of the target nucleic acid by generally known methods and/or commercially available extraction kits may be made in advance. In addition, when a double-stranded nucleic acid (DNA or RNA) has to be detected a strand separation has to be made in advance. This strand separation may be conducted e.g. chemically, with UV-light or by heat treatment.

As used herein, "nucleic acid" or "polynucleic acid" refers to the order or sequence of nucleotides along a strand of nucleic acids. The nucleic acid sequence may be single-stranded or double-stranded or contain portions of both double-stranded and single-stranded sequences. The nucleic acid sequence may be composed of DNA, both genomic and cDNA, RNA or DNA/RNA hybrid.

As used herein, "molecular beacons", or "(molecular beacon) probes", are oligonucleotide hybridization probes that can report the presence of specific nucleic acids in homogenous solutions. Molecular beacons are hairpin-shaped molecules with an internally quenched fluorescent dye whose fluorescence is restored when they bind to a target nucleic acid sequence. This is a novel nonradioactive method for detecting specific sequences of nucleic acids. They are useful in situations where it is either not possible or desirable to isolate the probe-target hybrids from an excess of the hybridization probes.

A typical molecular beacon probe is 25-40 nucleotides long. The middle nucleotides are complementary to the target DNA or RNA and do not base pair with one another, while the five to seven nucleotides at each terminus are complementary to each other rather than to the target DNA. A typical molecular beacon structure can be divided in 4 parts: 1) loop, an 18-30 base pair region of the molecular beacon that is complementary to the target sequence; 2) stem formed by the attachment to both termini of the loop of two short (5 to 7 nucleotide residues) oligonucleotides that are complementary to each other; 3) 5' fluorescent dye at the 5' end of the molecular beacon, a fluorescent dye is covalently attached; 4) 3' quencher (non-fluorescent) dye that is covalently attached to the 3' end of the molecular beacon. When the beacon is in closed loop shape, the quencher resides in proximity to the fluorescent dye, which results in quenching the fluorescent emission of the latter. If the nucleic acid to be detected is complementary to the strand in the loop, the event of hybridization occurs. The duplex formed between the nucleic acid and the loop is more stable than that of the stem because the former duplex involves more base pairs. This causes the separation of the stem and hence of the fluorescent dye and the quencher. Once the fluorescent dye is no longer next to the quencher, illumination of the hybrid with light results in the fluorescent emission. The presence of the emission reports that the event of hybridization has occurred and hence the target nucleic acid sequence is present in the test sample. The fluorescent dye of the molecular beacon may be any suitable fluorescent dye, for example ABI dyes (e.g. FAM™, HEX™, TET™, JOE™, ROX™, CAL Fluor™ Red 610), cyanine dyes (e.g. Yakima Yellow or ATTO) or molecular dyes (e.g. ALEXA-fluor or BODIPY dyes). The quencher of the molecular beacon may be any suitable quencher, for example TAM, BHQ1, BHQ2, DAB, Eclip, BBQ650. Molecular beacons are synthetic oligonucleotides whose preparation is well documented in the literature (Tyagi S; Kramer FR (1996). "Molecular beacons: probes that fluoresce upon hybridization". Nat. Biotechnol. 14 (3): 303-308). Further, the molecular beacon probe design is within the skill of a molecular biologist and there are many bioinformatics tools for this purpose available (e.g. Beacon Designer™). In addition, molecular beacon probes are commercially available for many target sequences (e.g. Eurofins Genomics, Ebersberg, Germany; Integrated DNA Technologies, Inc., Coralville, Iowa, USA.

In the context of this invention, "hybridization" means hydrogen bonding, which may be Watson-Crick, Hoogsteen or reversed Hoogsteen hydrogen bonding, between complementary nucleoside or nucleotide bases. Hybridization takes place under stringent or non-stringent conditions. As used herein, "stringent" refers to the conditions, i.e. temperature, buffer composition or ionic strength under which hybridization between polynucleotides occurs. These conditions depend mainly on the composition and complexity of the target nucleic acid and length of the probe. For the hybridization temperature conditions the "Tm" (melting temperature) of the nucleic acids has to be considered. "Tm" means under specified conditions the temperature at which half of the nucleic acid sequences are disassociated and half are associated. Generally, suitable hybridization conditions may be easily determined by a person skilled in the art.

"Complementary" as used herein, refers to the capacity for pairing between two nucleotides. For example, if a nucleotide at a certain position of an oligonucleotide is capable of hydrogen bonding with a nucleotide at the same position of a DNA or RNA molecule, then the oligonucleotide and the target DNA or RNA are considered to be complementary to each other at that position. For example, the sequence 5'-A-C-T-3' is complementary to the sequence 3'-T-G-A-5'. Complementarity may be partial, in which only some of the nucleotides match according to base pairing, or complete, where all the nucleotides match according to base pairing. For purposes of the present invention "substantially complementary" refers to 90% or greater identity over the length of the target base pair region. The complementarity can also be 45, 50, 60, 70, 75, 80, 85, 90, 91, 92, 93, 94, 95, 96, 97, 98, 99, or 100% complementary, or any amount below or in between these amounts. In other words, the oligonucleotide and the target DNA or RNA are complementary to each other when a sufficient number of corresponding positions in each molecule are occupied by nucleotides which can build a hydrogen bond with each other. Thus, "specifically hybridizable" and "complementary" are terms which are used to indicate a sufficient degree of complementarity or precise pairing such that stable and specific binding occurs between the probe and the DNA or RNA target. It is understood in the art that the probe does not need to be 100% complementary to that of its target nucleic acid to be specifically hybridizable. "Oligonucleotide" refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly.

"Subject" or "patient" as used herein refers to a mammalian individual, including murines, cattle, simians and humans. Preferably, the patient is a human.

"Sample" as used herein refers to a biological sample encompassing liquid and solid samples. Liquid samples encompass saliva, sputum, sweat, urine, nasal secretion, bronchoalveolar lavage fluid, laryngo-pharyngeal scrape test, vaginal secretion, blood liquids (e.g. serum, plasma) and cerebrospinal fluid (CSF). Solid samples encompass tissue samples such as tissue cultures or biopsy specimen. A preferred patient's sample is sputum. The patient's sample will be collected with the disposable pipette as described below.

In a particular preferred embodiment of the present invention, the method is used for detecting a virus in a patient's sample. "Virus" or "viral" means any single- or double-stranded DNA or RNA virus. In particular, Human Immunodeficiency Virus (HIV), Zika, MERS-Corona, SARS-CoV-1, Sars-CoV-2, Hepatitis B Virus (HBV), Hepatitis C Virus (HCV), Human Lyphotropic Virus-type 1-4 (HTLV-1 - 4), Epstein-Barr Virus (EBV), Human Papilloma Virus (HPV), Influenza A, B or C, Non-Influenza Respiratory Viruses (NIRVs, e.g. respiratory syncytial virus, parainfluenza virus, rhinovirus, metapneumovirus), norovirus, Ebola virus, Reovirus, Herpes virus (simplex 1, 2 + B, 5, 6, 7, 8), Pox virus, FSME virus, Dengue virus, Rubivirus, Varizella zoster virus and Cytomegalievirus.

In the method according to the invention, the carrier element is preferably a polymer, in particular a liquid swelling polymer. Examples are agarose gel, hydrogel, polyamide gel or polyester gel. In this method, the polymer has several functions: (1) binding a large number of probe molecules, (2) absorbing target molecules from the sample, (3) purification of the target molecules and (4) immobilizing the probes around an optical fiber so that the fluorescence appearing in result of hybridization has larger probability of coupling to the optical fiber. Agarose is well known in molecular biology laboratory application, and in particular easy to adapt for binding and absorbing specific molecules.

In a method according to the invention, the carrier element preferably contains at least 20 *nmol*/*l,* preferably at least 2.5 *µmol*/*l* of the probe. In this method, almost every target molecule migrating into the carrier element is immediately in contact with a probe molecule that is ready for hybridizing.

In a method according to the invention, the target nucleic acid may in particular be a virus RNA. This method provides for screening large numbers of patients for infection, in an extremely reduced time scale, compared to the state of the art. The virus may in particular be a coronavirus, further in particular SARS-CoV-2. The method is thus applicable in a recent world-wide pandemic situation.

In a method according to the invention, the excitation light may in particular be a laser beam. This method allows for limiting the excitation light to the excitation wavelength of the fluorescent dye, and thus for avoiding side effects induced by other wavelengths.

In a method according to the invention, before irradiating the carrier with the excitation light, preferably the same is heated to at least 65 °*C*. Either in addition, or in an alternative method according to the invention, before irradiating the carrier with the excitation light, the same is exposed to ultraviolet radiation. Both heating and exposing to UV radiation removes the capsid around the target nucleic acid and increases the release of the target nucleic acid from the sample. In case of a double-stranded nucleic acid it may also lead to denaturation.

In a method according to the invention preferably a peak wavelength of the emitted light is defined that is specific to a hybrid of the target and the probe, and if the emitted light has the peak wavelength, it is concluded that the target is in the sample. This method is based on the finding that each target-probe hybrid has a unique wavelength of emitted light, and allows for identifying any known target, in a patient's sample. In particular, the peak wavelength *λₑₘ* may be calculated from *h* · *c* / *λₑₘ = h · c*/*λ_{ref}* - *E₀,* wherein *λ_{ref}* is a reference emission of the fluorescent dye without hybridization, *h* is the Planck constant, *c* is the speed of light and *E₀* is the hybridization energy of the target-probe hybrid, as known from Santalucia J Jr, Proc. Natl. Acad. Sci. USA 95 (1998) and calculated in terms of Nearest-Neighbor (NN) model, where change of the energy is 0.0243 *eV.*

The present invention further concerns an analyzing device for a method according to the invention, having a light source for the excitation light, and a microprocessor that is arranged for automatically receiving data from the detector, and for concluding that the target is in the sample. This device allows for automatic execution of the method according to the invention.

A device according to the invention preferably has a beam splitter with high pass optical filter, or a dichroic mirror between the carrier element and the detector which eliminates the excitation light from a signal to be measured by the detector. In this device, only the emitted light is protruding to the detector.

In a device according to the invention, preferably the optical element is an optical fiber. In an alternative device according to the invention, the optical element is a glass window. Optical fibers and glass windows are commonly known and readily available for building a device according to the invention.

A device according to the invention preferably has at least one focusing element between the light source and the carrier element which delivers the excitation light onto the carrier element. In this device, the excitation effect onto the probe is improved.

A device according to the invention preferably has at least one beam collimating element between the carrier element and the optical element which collects the emitted light to the optical element. In this device, detection of the emitted light is improved.

The present invention further concerns a sampling unit for adding into an analyzing unit whereby the sampling unit and the analyzing unit form such analyzing device, the sampling unit having the carrier element and the optical element, and a container for collecting the patient's sample, and for isolating the sample from the environment, wherein the container in particular is a pipette. This unit allows for secure handling of infectious matter. The sampling unit may be a low-cost one-time disposable product.

### Best Mode Examples

Preferred embodiments of the invention are described with reference to the figures, showing
- in fig. 1a: an first analyzing device according to the invention,
- in fig. 1b: the analyzing unit of the first device,
- in fig. 2: an analyzing unit of another device according to the invention,
- in fig. 3: a second analyzing device according to the invention, and
- in fig. 4.: fluorescence spectra of samples with and without target RNA

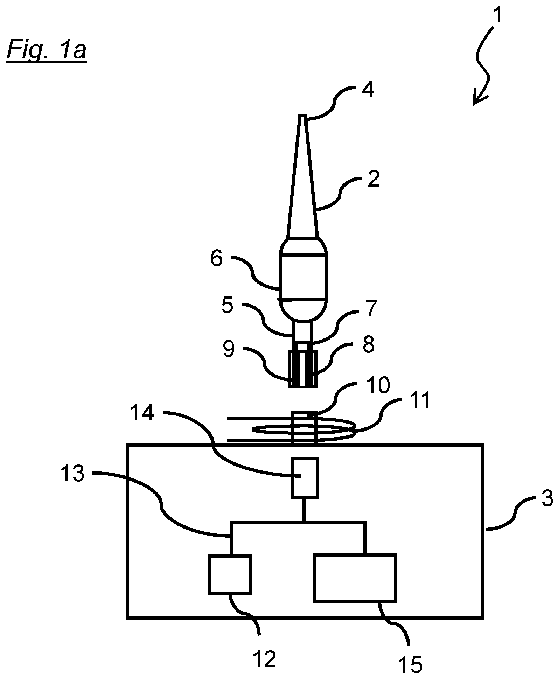

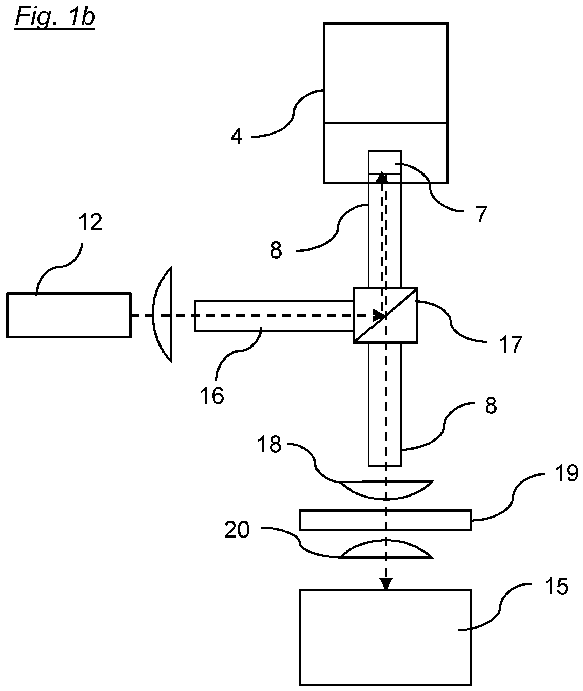

The first analyzing device 1 shown in fig. 1a comprises a disposable sampling unit 2 and an analyzing unit 3, which is in detail shown in fig. 1b.

The sampling unit 2 is formed as a pipette, and comprises an extraction nozzle 4, a reaction chamber 5 surrounded by flexible walls 6, a carrier element 7 inside the reaction chamber 5, an optical element 8 commutating the carrier element 7 with the analyzing unit 3, and a ferrule 9 for aligning the optical element 8 to the same.

The extraction nozzle 4 has a single use liquid tide cap (not shown) for locking the nozzle 4 after extraction of the patient's sample (not shown) by pressing and releasing the walls 6. The carrier element 7 is a block of 20 *nl* of polymerized agarose, which carries 2.5 *µmol*/*l* of beacon probe molecules labeled with a fluorescent dye, and designed to hybridize with SARS-CoV-2 RNA.

To provide sterility in delivery, the sampling unit 2 is packed individually in clean plastic (not shown). In another analyzing device according to the invention, the sampling unit 2 is packed in a similar bag or container. The sampling unit 2 has respective marks and labels that the control unit may recognize what kind of test to perform. In another analyzing device according to the invention, the sampling unit 2 has an RFID chip. The analyzing unit 3 also can send request into a data base in order to validate the sampling unit 2 (e.g. expiration date, was the tester used before).

The analyzing unit 3 comprises an insertion slot 10 for the sampling unit 2 with a heating element 11 for speeding-up of the hybridization, a light source 12 commutated with a Y-fiber 13 and an optical fiber connector 14. The connector 14 serves for efficient transfer of light power from the light source 12 to the optical element 8, and to the carrier element 7. The second arm of the Y-fiber 13 redirects fluorescence from the carrier element 7 to a detector 15, namely a spectrometer In other embodiments of the invention, the analyzing unit 3 is equipped with a system enabling detection when new pipette inserted into the slot 10, and/or with a computer or other device capable to readout spectra from detector 15 and perform inline mathematical analysis of measured spectra, and/or other optical elements for improving quality of the RNA/DNA detection, and/or an automatic shutter to protect the slot 10 from contamination, and/or an additional light source 12 for detecting presence of a sample, in the container.

In order to eliminate dust contamination of the open end of the optical element 8, a protection cup is used. The open side of the sampling unit 2 is protected with a cup after collection of the patient's sample (both cups not shown).

For collecting a sample, an operator of the analyzing device 1 (the patient himself or health personnel) inserts the sampling unit 2 into the patient's mouth, presses with fingers on the walls 6 of the reaction chamber 5 and releases until few drops of sputum are sucked in, and seals the nozzle 4 with the respective cup. For detecting SARS-CoV-2 in the patient's sample, the operator removes the protection cup from the optical element 8, and inserts the sampling unit 2 into the slot 10, and initiates analysis procedure by pressing a start button (not shown) on the analyzing unit 3. After obtaining the result, the operator removes the sampling unit 2 from the slot 10 and puts it into a gated bin for following disintegration/disinfection of the sampling unit 2 and its content.

The light source 12 emits laser light with 532 *nm.* The light source 12 emits laser light into an excitation arm 16 of the Y-fiber 13. A beam splitter 17 reflects 10% of the laser power to the carrier element 7 through the optical element 8. Light emitted by the fluorescent dye is collected by the optical element 8. After the beam splitter 17, 90 % of the emitted light is collected by a focusing and beam collimating element 18, namely a lens. Scattered laser light is filtered from the emitted light by a high pass filter element 19, and collected on an entrance slit of the detector 15 by a further focusing lens 20.

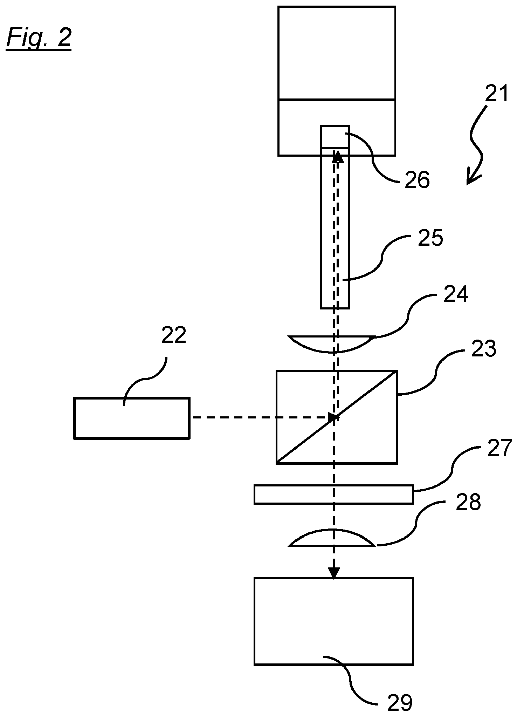

Fig. 2 shows a second analyzing unit 21 of yet another analyzing device according to the invention (not shown). In the analyzing unit 21, 10 % of laser light emitted from a light source 22 similar to the first analyzing unit 3 is reflected by a beam splitter 23 to a focusing and beam collimating element 24, namely a lens. In another analyzing device according to the invention, the focusing and beam collimating element 24 is an optical fiber collimator. An optical element 25 delivers the light to the carrier element 26. Light emitted by the fluorescent dye (at least part of it) is collected with the optical element 25 and delivered to the focusing and beam collimating element 24. 90% of the emitted light passes through the beam splitter 23 and further through a high pass optical filter element 27. A further lens 28 focuses the emitted light to a detector 29. In another configuration the beam splitter 23 and the optical fiber 27 can be replaced with respective dichroic mirror, which has to reflect the wavelength emitted by the laser and transmit fluorescent light.

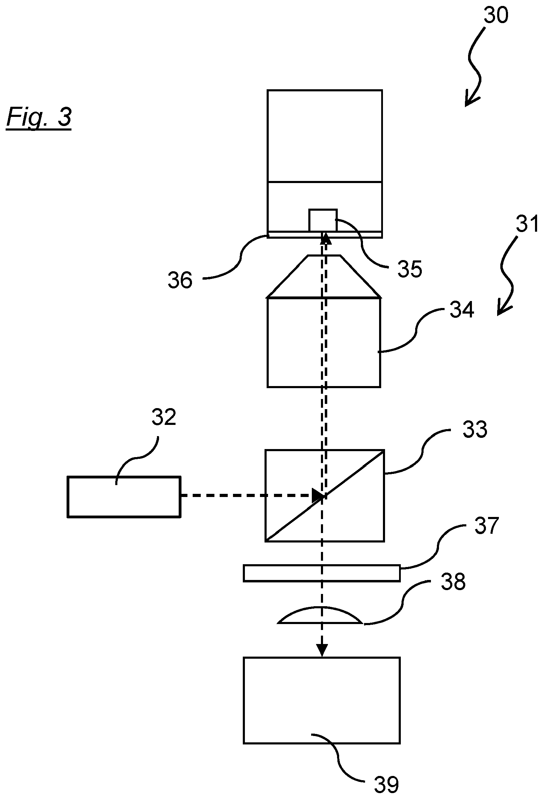

Fig. 3 shows a second analyzing device 30 according to the invention. In an analyzing unit 31 of the second analyzing device 30, 10 % of laser light emitted from a light source 31 again similar to the first analyzing unit 3 is reflected by a beam splitter 33 towards a focusing and beam collimating element 34, namely a planar micro objective. The focusing and beam collimating element 34 focuses excitation light on a carrier element 35 through an optical element 36, namely a glass window. Light emitted by the fluorescent dye passes through the optical element 36 and is collected by the micro-objective. 90% of the emitted light passes through the beam splitter 33 and further through a high pass optical filter element 37. A further lens 38 focuses the emitted light to a detector 39.

The invention is further described with reference to the following Example:

### Example 1: Micro-Fluorescence Measurement

Agarose powder was weighted and mixed with a buffer solution (e.g. 0.1 M phosphate buffer, pH 6.5 or 20 mM Tris-HCI buffer, pH8.0), so that a 4.0% agarose gel was obtained after heating of the mixture. The mixture was heated up to 90°C and carefully mixed for about 10 min. 10 µL of the gel was retrieved with a pipette and inserted into a cuvette. The cuvette with agarose gel was kept at 60-70°C.

10 µL of a solution 20 mM Tris-HCl buffer, pH8.0, containing 2.5 µM/l of Corona E-Beacons labeled with Rhodamine 6G and quenched with BMN-Q620 (biomers.net GmbH Ulm, Germany) was retrieved and inserted into the cuvette containing the 10 µL of agarose gel.

The Corona E-Beacon had the sequence:
Rhodamine 6G-ACACTAGCCATCCTTACTGCGCTTCG-BMNQ620

This results in a final concentration of around 2% agarose. The mixture was mixed for about 30 seconds and was deposited with help of another pipette on the bare end of an optical fiber. The resulting polymer layer had a volume of much less than 20 nL.

Four test samples were analyzed:
1. Extracted RNA (in 20 mM Tris-HCI buffer, pH 8.0) obtained from a human patient and previously analyzed (passed through) with PCR. The test sample is characterized by absence of the virus RNA - #0 Negative
2. Extracted RNA (in 20 mM Tris-HCI buffer, pH 8.0) obtained from a human patient and previously analyzed (passed through) with PCR. The test sample had low concentration (about 100 targets per reaction) of the virus RNA - #1 Positive
3. Extracted RNA (in 20 mM Tris-HCI buffer, pH 8.0) obtained from a human patient and previously analyzed (passed through) with PCR. The test sample had higher concentration (about 3,000) of the virus RNA - #2 Positive
4. Extracted RNA (in 20 mM Tris-HCI buffer, pH 8.0) obtained from a human patient and previously analyzed (passed through) with PCR. The test sample had very high concentration (about 3,700,000) of the virus RNA - #3 Positive

The optical fiber with the polymer layer was immersed into the "#0 Negative" test sample. The fluorescence spectra of the "#0 Negative" sample were measured for 4-5 min.

Another optical fiber with the polymer layer with exactly the same concentration of molecular beacons was one after another immersed into #1 Positive, #2 Positive and #3 Positive and the fluorescence spectra were each collected for about 4-5 min.

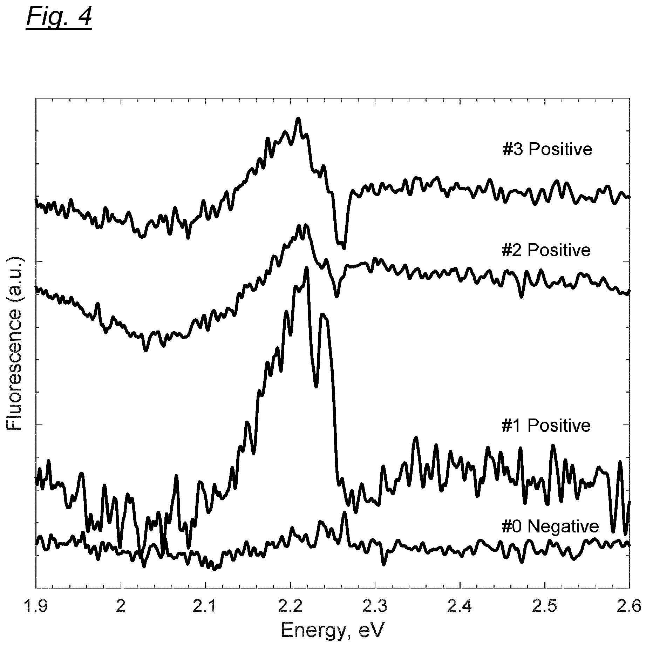

The result of the fluorescence measurement is shown in Fig. 4.

As can be seen from Figure 4, the fluorescence measured in the Positive samples is at 2.208 *eV.* In accordance with the molecular beacons as probes specification they were labeled with Rhodamine 6G with maximum emission at 2.232 eV. The difference between expected and observed fluorescence is 0,0243 eV could be attributed to lowering of energy due to primer - target hybridization, so E1 > E2.

In terms of temperature, probe - target hybridization leads to lowering of the free energy of RNA-Molecular Beacon hybrid (ΔG) = -0.558 kcal/mol.

In the figures are
- 1: Analyzing device
- 2: Sampling unit
- 3: Analyzing unit
- 4: Extraction nozzle
- 5: Reaction chamber
- 6: Flexible wall
- 7: Carrier element
- 8: Optical element
- 9: Ferrule
- 10: Insertion slot
- 11: Heating element
- 12: Light source
- 13: Y-fiber
- 14: Optical fiber connector
- 15: Detector
- 16: Excitation arm
- 17: Beam splitter
- 18: Focusing/Beam collimating element
- 19: High pass filter element
- 20: Lens
- 21: Analyzing unit
- 22: Light source
- 23: Beam splitter
- 24: Focusing/Beam collimating element
- 25: Optical element
- 26: Carrier element
- 27: Filter element
- 28: Lens
- 29: Detector
- 30: Analyzing device
- 31: Analyzing unit
- 32: Light source
- 33: Beam splitter
- 34: Focusing/Beam collimating element
- 35: Carrier element
- 36: Optical element
- 37: Filter element
- 38: Lens
- 39: Detector

## Claims

1. In-vitro method for detecting a target nucleic acid in a patient's sample, comprising the following steps:
a. Bringing the sample in contact with a probe, that is a molecular beacon labeled with a fluorescent dye, and can hybridize with the target, then
b. Irradiating the probe with light exciting the fluorescent dye, then
c. Observing the sample with a detector (15, 29, 39), and then
d. If the detector (15, 29, 39) detects light emitted by the probe, concluding that the target is in the sample,
***Characterized by**,* prior to bringing in contact with the sample, immobilizing the probe in a carrier element (7, 26, 35), wherein the carrier element (7, 26, 35) is in contact with an optical element (8, 25, 36) which lets the emitted light pass to the detector (15, 29, 39).

2. Method according to the preceding claim, ***characterized in that*** the carrier element (7, 26, 35) is a polymer.

3. Method according to the preceding claim, ***characterized in that*** the carrier element (7, 26, 35) contains at least 20 *nmol*/*l,* preferably at least 2.5 *µmol*/*l* of the probe.

4. Method according to one of the preceding claims, ***characterized in that*** the excitation light is a laser beam.

5. Method according to one of the preceding claims, ***characterized by**,* before irradiating with the excitation light, heating the carrier element (7, 26, 35) to at least 65 °*C*, and/or exposing the carrier element (7, 26, 35) to ultraviolet radiation.

6. Method according to one of the preceding claims, ***characterized by*** defining a peak wavelength of the emitted light that is specific to a hybrid of the target and the probe, and if the emitted light has the peak wavelength, concluding that the target is in the sample.

7. Analyzing device (1, 30) for a method according to one of the preceding claims, ***characterized by*** a light source (12, 22, 31) for the excitation light, and a microprocessor that is arranged for automatically receiving data from the detector (15, 29, 39), and for concluding that the target is in the sample.

8. Analyzing device (1, 30) according to the preceding claim, ***characterized by*** a beam splitter with high pass optical filter, or a dichroic mirror between the carrier element (7, 26, 35) and the detector (15, 29, 39) which eliminates the excitation light from a signal to be measured by the detector (15, 29, 39).

9. Analyzing device (1, 30) according to one of claims 7 and 8, ***characterized in that*** the optical element (8, 25, 36) is an optical fiber, or a glass window.

10. Analyzing device (1, 30) according to one of claims 7 to 9, ***characterized by*** at least one focusing element (18, 24, 34) between the light source (12, 22, 32) and the carrier element (7, 26, 35) which delivers the excitation light onto the carrier element (7, 26, 35).

11. Analyzing device (1, 30) according to one of claims 7 to 10, ***characterized by*** at least one beam collimating element (18, 24, 34) between the carrier element (7, 26, 35) and the optical element (8, 25, 36) which collects the emitted light to the optical element (8, 25, 36).

12. Sampling unit (2) for adding into an analyzing unit (3, 21, 31) whereby the sampling unit (2) and the analyzing unit (3, 21, 31) form an analyzing device (1, 30) according to one of claims 7 to 11, ***characterized in that*** the sampling unit (2) contains the carrier element (7, 26, 35) and the optical element (8, 25, 36), and a container for collecting the patient's sample, and for isolating the sample from the environment, wherein the container in particular is a pipette.
